Europäisches Patentamt

European Patent Office

Office européen des brevets

(11) Publication number: **0 173 110**
A2

# EUROPEAN PATENT APPLICATION

(21) Application number: 85109713.9

(22) Date of filing: 02.08.85

(51) Int. Cl.⁴: **G 02 B 6/40**

(30) Priority: 23.08.84 US 643502

(43) Date of publication of application: 05.03.86 Bulletin 86/10

(84) Designated Contracting States: BE CH DE FR GB IT LI SE

(71) Applicant: WESTINGHOUSE ELECTRIC CORPORATION, Westinghouse Building Gateway Center, Pittsburgh Pennsylvania 15222 (US)

(72) Inventor: King, William Earl, 9262 Wedgewood Drive, Pittsburgh Pennsylvania 15239 (US)

(74) Representative: Patentanwälte Dipl.-Ing. R. Holzer Dipl.-Ing. (FH) W. Gallo, Philippine-Welser-Strasse 14, D-8900 Augsburg (DE)

(54) Fiberoptic light guide.

(57) A fiberoptic light guide is described which includes a fiberoptic bundle of pure silica to provide relatively low light energy losses per unit length. To improve the light acceptance characteristics of the fiberoptic bundle, a relatively short second fiberoptic bundle having excellent light reception characteristics due to a high numerical aperture is coupled to the first fiberoptic bundle by means of a higher efficiency fiberoptic coupling. The fiberoptic light guide, by possessing excellent light reception characteristics, as well as relatively low light energy losses per unit length, may be efficiently used with commercially available light sources such as incandescent lamps in a variety of medical and industrial applications for the visual inspection of remote, relatively inaccessible areas.

0173110

1

# FIBEROPTIC LIGHT GUIDE

## BACKGROUND OF THE INVENTION

### Field of the Invention:

The present invention relates in general to an optical light guide, and more particularly, to a fiberoptic light guide for transmitting light energy of the monochromatic or polychromatic type from a source thereof to a remote relatively inaccessible area to be visually inspected upon illumination by the manual or robotic manipulation of the distal end of the light guide. Specifically, the fiberoptic light guide described herein is constructed and arranged to include a flexible light transmissive element of substantial length composed of pure silica and featuring minimum light energy loss per unit length and a coupled light-receiving portion of relatively short length having enhanced light reception characteristics via a high numerical aperture.

### Description of the Prior Art:

The usage of fiberoptic bundles for light and image transmission continues to rapidly multiply. A preferred application of fiberoptic bundles is the fiberscope, which receives and transmits an image from relatively inaccessible areas for remote visual inspection. Typical applications are too numerous to comprehensively mention, but these applications extend throughout both the medical and industrial fields, for example, examination of internal organs of a human body, diagnostic inspection of internal combustion engines, inspection of nuclear power plants, and the like. In such applications, a second fiberoptic bundle, the light guide, transmits light to the area to be viewed by the fiberscope. Therefore, the effectiveness of the fiberscope depends, in part, on the amount of light which can be transmitted through the light guide.

Fiberoptic bundles of pure silica, i.e., quartz, are desirable for use in transmission of light and image due to their low light energy loss characteristics along their transmission length. However, these pure silica fiberoptic bundles have a relatively low numerical aperture. It is well-known that a low numerical aperture precludes the ability of the fiberoptic bundle to accept or receive light energy beyond a predetermined acceptance cone for transmission. For a known cross-sectional area, a fiberoptic bundle having a numerical aperture of 0.4 has a calculated increase in light energy acceptance of about 475% over a similar fiberoptic bundle having a numerical aperture of only 0.2. Thus, the low numerical aperture of these pure silica fiberoptic bundles, although possessing low light energy loss per unit length, significantly reduces the amount of light energy available to illuminate the area to be visually inspected and thus, ultimately reduces the effectiveness of the fiberscope.

The technological advances in overcoming this disadvantage of using pure silica fiberoptic bundles having a low numerical aperture, have been primarily related to the fiberscope. In this regard, improvements in the light energy acceptance of these pure silica fiberoptic bundles have been obtained through the use of a complex system of lenses. Such complex lens systems have also found limited use in improving the light acceptance characteristics of fiberoptic light guides as evidenced by U.S. Patent No. 3,669,524. However, these lens systems, in addition to being complex, are also expensive for many routine applications, for example, in general medical examinations and the

3

like. Another solution to the foregoing problem of light energy acceptance has been contemplated by the use of lasers which emit a narrow beam of monochromatic light. However, in addition to the fact that lasers are cost prohibitive for many routine applications as noted, monochromatic light cannot be used for color imaging which therefore limits the application of the fiberoptic light guide.

Accordingly, it can be appreciated that there is an unsolved need for a fiberoptic light guide which is inexpensive and simple to manufacture and possesses enhanced light energy acceptance characteristics, while possessing low light energy loss per unit length.

## SUMMARY OF THE INVENTION

It is broadly an object of the present invention to provide a fiberoptic light guide suitable for the low light energy loss transmission of monochromatic or polychromatic light from a source thereof to a relatively inaccessible area for remote visual inspection, which overcomes or avoids one or more of the foregoing disadvantages resulting from the use of the above-mentioned prior art light guides, and which fulfills the specific requirements of such a fiberoptic light guide for use with conventional inexpensive light sources. Specifically, it is within the contemplation of one aspect of the present invention to provide a fiberoptic light guide of pure silica which increases the ability of the fiberoptic bundle to accept or receive light energy for transmission while maintaining low light energy losses per unit length.

Described herein is a fiberoptic light guide which is inexpensive to manufacture and is of simple construction, which is adapted for manual or robotic manipulation into relatively inaccessible areas for the remote visual inspection thereof upon illumination of the area by the fiberoptic bundle, which employs high efficiency coupling techniques to minimize light energy losses, which increases the light energy accepted by the fiberoptic bundle when employing pure silica fiberoptic bundles having a relatively low numerical aperture, and which is adapted for use in a variety of applications such as in the medical and industrial field.

The invention in its broad form comprises a fiberoptic light guide comprising a first fiberoptic fiber element of known aperture for receiving light from a light source, a second fiberoptic fiber element assembled for receiving said light from said first fiber, said first fiber having a numerical aperture greater than that of said second fiber, and coupling means for coupling said first and second fibers in light transmissive relationship.

In accordance with one embodiment of the present invention, there is provided fiberoptic light guide constructed of a first fiberoptic fiber for receiving light from a source thereof, a second fiberoptic fiber for receiving the light from the first fiber, the first fiber having a numerical aperture greater than that of the second fiber, and coupling means for coupling the first and second fibers in light transmissive relationship.

In accordance with another embodiment of the present invention, there is provided a fiberoptic light guide constructed of a first fiberoptic fiber for receiving light from a source thereof, a second fiberoptic fiber consisting essentially of silica for receiving the light from the first fiber, the first fiber having a numerical aperture greater than and a length shorter than that of the second fiber, and coupling means for coupling the first and second fibers in light transmissive end-to-end relationship.

In accordance with the above-mentioned embodiments, the first and second fiberoptic fibers include a plurality of such fibers arranged in a bundle, the numerical aperture of the first fibers being greater than about 0.4, and the numerical aperture of the second fibers being less than 0.2.

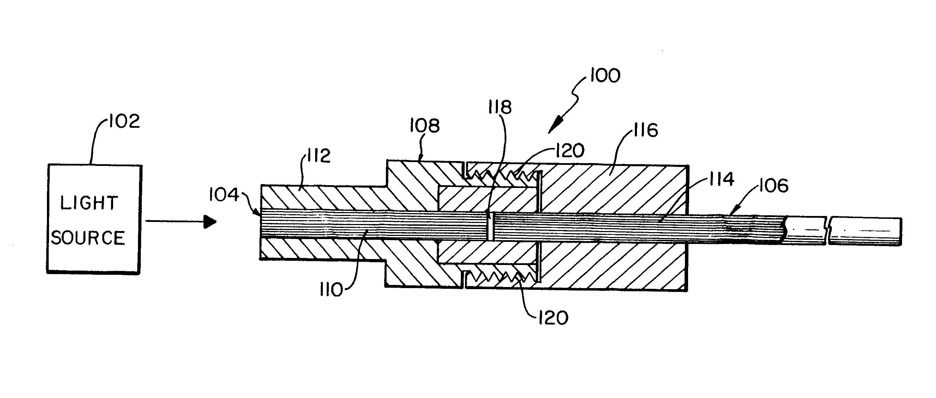

A more detailed understanding of the invention may be had from the following description of a preferred embodiment given by way of example and to be studied in conjunction with the accompanying drawing wherein the sole drawing discloses a fiberoptic light guide constructed of a first bundle of fiberoptic fibers having a relatively low numerical aperture coupled, via a high efficiency coupling, in end-to-end relationship with a second bundle of fiberoptic fibers having a relatively high numerical aperture.

## DESCRIPTION OF THE PREFERRED EMBODIMENTS

Referring now to the sole drawing, wherein like reference numerals represent like elements, there is shown a fiberoptic light guide generally designated by reference numeral 100. The light guide 100, as thus far described, is characterized by its excellent light reception characteristics while having minimal light energy losses per unit length. These characteristics of the light guide 100 render it suitable for use with a conventional inexpensive light source 102, such as an incandescent lamp which provides a source of polychromatic light. However, other sources of polychromatic light, as well as monochromatic light, may be employed.

As shown, the light guide 100 is constructed of a first fiberoptic bundle 104 and a second fiberoptic bundle 106 coupled in light transmissive end-to-end relationship by a high efficiency fiberoptic coupling 108. The first fiberoptic bundle 104 includes a plurality of individual fiberoptic fibers 110 of the multi-component type having a relatively high numerical aperture and therefor excellent light reception characteristics. For example, suitable fibers 110 for use with the present invention include those formed of sodium borosilicate and lead silicate, each having a numerical aperture of 0.5. The number of individual fibers 110 being arranged in the first fiberoptic bundle 104 can number from 1 to 50,000, each having an outside diameter in the range of from 1 to 1,000 microns.

The particular composition of the fibers 110, the number of such fibers arranged in the first fiberoptic bundle 104, and the size of the individual fibers will vary depending upon the application to which the light guide 100 is to be used, as well as the characteristics of the available light source 102. In general, the larger the diameter of the fibers 110 and the higher the numerical aperture, the more light that can be received by the first fiberoptic bundle 104 from the light source 102. However, as the material composition of these fibers 110 results in their having a relatively high light energy loss per unit length, it is desirable that the length of the first fiberoptic bundle 104 be as short as possible, and preferably less than 7.6 centimeters, although other lengths may be used. It is generally only required that the length of the first fiberoptic bundle 104 be sufficient to be engaged by the forward end 112 of the fiberoptic coupling 108.

As the first fiberoptic bundle 104 has a high light energy loss per unit length, it is undesirable to extend the bundle for any significant length, such as from the light source 102 to the remote area to be visually inspected. To this end, the fiberoptic light guide 100 includes a second fiberoptic bundle 106 including a plurality of individual fiberoptic fibers 114 having a composition of pure silica, e.g., quartz and those materials having similar optical properties, containing generally less than 5 to 10 parts per billion of impurities. These pure silica fibers 114 have a relatively low numerical aperture of 0.2 or less and therefore possess only moderate light reception characteristics. On the other hand, these pure silica fibers 114 have low light energy loss per unit length, and are therefore suitable for transmitting light over a substantial distance from a source thereof to remote areas to be visually inspected. The pure silica fibers 114 can number from 1 to 50,000 in the second fiberoptic bundle 106, each having an outside diameter in the range of from 1 to 1,000 microns, and up to 200 meters long. The specific number of the pure silica fibers 114 contained within the second fiberoptic bundle 106, their respective diameters and length are dictated by the specific application of the light guide 100, the nature of the light source 102 and the location of the area for remote visual inspection. However, it is again noted that the larger the diameter of the pure silica fibers 114, as well as the number of such fibers within the second bundle 106, the more light that can be transmitted through the second fiberoptic bundle from the light source 102. The individual fiberoptic fibers 110, 114 which make up the first and second fiberoptic bundles 104, 106 are commercially available for instance from Diaguide of Orangeburg, New York.

The first and second fiberoptic bundles 104, 106 are coupled together in light transmissive end-to-end relationship by means of a fiberoptic coupling 108. The coupling 108 is a multiple core connector of the high efficiency type available from Diaguide and designated as type D-60P/D-60F. The coupling 108 includes a male forward end 112 which receives the first fiberoptic bundle 104 as previously described. A female rearward end 116 is provided which receives the second fiberoptic bundle 106 for coupling the fiberoptic bundles 104, 106 in light transmissive end-to-end relationship. The male forward end 112 and female rearward end 116 are removably secured together by circumscribing internal and external threaded portions 120. The first and second fiberoptic bundles 104, 106 may be butted together in end-to-end relationship, or may include a space 118 provided therebetween. The space 118 can be filled with water or other manufacturer's proprietary liquid which increase the light energy coupling efficiency between the first and second fiberoptic bundles 104, 106. Although a specific construction and designated type of fiberoptic coupling 108 has been described, other types of fiberoptic couplings may be employed.

The present invention provides a fiberoptic light guide 100 constructed of a short multi-component first fiberoptic bundle 104 having a relatively large numerical aperture for increased light energy acceptance coupled, by means of a high efficiency fiberoptic coupling 108, to a pure silica second fiberoptic bundle 106 having relatively low light energy losses per unit length. The relative short length of the first fiberoptic bundle 104 will limit light energy losses to under 5% of the light energy received from the light source 102. The high efficiency fiberoptic coupling 108 is capable of coupling light energy from the first fiberoptic bundle 104 to the second fiberoptic bundle 106 with a light energy loss of less than about 10%. Although the second fiberoptic bundle 106 has a relatively low numerical aperture, substantially all light energy leaving the first fiberoptic bundle will be within the acceptance cone of the pure silica second fiberoptic bundle 106. As previously indicated, the use of a first fiberoptic bundle 104 having a numerical aperture of 0.4 coupled to a second fiberoptic bundle having a numerical aperture of 0.2 results in a calculated 475% more light energy being introduced into the fiberoptic light guide 100. Taking into consideration the two additional light energy loss factors, i.e., the first fiberoptic bundle 104 and fiberoptic coupling 108, there is still approximately a 406% gain in light energy available within the fiberoptic light guide 100. Accordingly, the construction of the fiberoptic light guide 100 in accordance with the present invention greatly enhances the ability of the fiberoptic light guide to efficiently use commercially available light sources 102 in a variety of applications.

Although the invention herein has been described with reference to particular embodiments, it is to be understood that these embodiments are merely illustrative of the principles and application of the present invention. It is to be understood that numerous modifications may be made in the illustrative embodiments and other arrangements may be devised without departing from the spirit and scope of the present invention as defined by the appended claims.

0173110

9

CLAIMS:

1. A fiberoptic light guide comprising a first fiberoptic fiber element of known aperture for receiving light from a light source, a second fiberoptic fiber element assembled for receiving said light from said first fiber, said first fiber having a numerical aperture greater than that of said second fiber, and coupling means for coupling said first and second fibers in light transmissive relationship.

2. The fiberoptic light guide of Claim 1 wherein said second fiber element has a length greater than that of said first fiber element.

3. The fiberoptic light guide of Claim 1 wherein said first and second fibers are coupled in end-to-end relationship.

4. The fiberoptic light guide of Claim 1 wherein said first element includes a plurality of said first fibers arranged in a bundle and each having a numerical aperture greater than that of said second fiber.

5. The fiberoptic light guide of Claim 1 further including a plurality of said second fibers arranged in a bundle and each having a numerical aperture smaller than that of said first fiber.

6. A fiberoptic light guide of Claim 1 wherein said second fiber element consists essentially of silica.

7. The fiberoptic light guide of Claim 1 wherein the numerical aperture of said first fiber element is greater than 0.4.

8. The fiberoptic light guide of Claim 1 wherein the numerical aperture of said second fiber is less than 0.2.

9. The fiberoptic light guide of Claim 8 wherein said first and second fibers have an outside diameter in a range of 1 to 1,000 microns.

10. The fiberoptic light guide of Claim 9 wherein said light source comprises a source of polychromatic light.

11. The fiberoptic light guide of Claim 9 wherein said light source comprises an incandescent lamp.

12. The fiberoptic light guide of Claim 9 wherein the length of said first fiber is less than 7.6 centimeters.

13. The fiberoptic light guide of Claim 9 wherein the length of said second fiber is up to 200 meters.

0173110